# EUROPEAN PATENT SPECIFICATION

(11) **EP 0 454 184 B1**
(45) Date of publication and mention of the grant of the patent: **31.08.1994**
(21) Application number: 91111304.1
(22) Date of filing: 03.04.1986
(51) Int. Cl.: A61F 7/03

(54) **Passive heating pad**
Passives Heizkissen
Coussin chauffant passif

(30) Priority: 04.04.1985 US 719986
(43) Date of publication of application: 30.10.1991
(62) Divisional of application: 86302479.0
(73) Proprietor: LASTRAP INC, Mississauga Ontario L5L 1T1 (CA)
(72) Inventor: Last, Anthony J., Oakville, Ontario L5L 1T1 (CA)
(74) Representative: Burford, Anthony Frederick

(56) References cited:
- GB-A- 2 074 452
- US-A- 2 595 328
- US-A- 2 909 176
- US-A- 2 911 974
- US-A- 3 092 110
- US-A- 4 470 417

## Description

The present invention relates to a therapeutic pad, which takes the form of a passive heating pad for therapeutic application.

This application is a division of our European patent application no. 86302479.0 filed April 3, 1986.

The use of heat as therapy for muscles or a group of muscles in the human body is a well-known technique. Prior art thermal pads employ some form of positive or external heating means, for example, electricity, chemical means, or steam or hot water. Typical prior art in this area are the following U.S. Patents: US-A-2,911,974, US-A-2,909,176, US-A-2,595,328, US-A-3,463,161 and US-A-3,901,225. As will become apparent from the discussion below, this prior art is not relevant to the present invention.

In one aspect of the invention, there is provided a passive heating pad for application to the body of an animal, including man, for the heat treatment of a muscle or group of muscles, for example, in the treatment of arthritis, comprising at least one flexible generally planar envelope dimensioned to engage an area of skin adjustment muscle tissue to be heated and flexible to snugly engage the skin area, characterized in that said envelope is filled with a permanently-liquid material having a viscosity in the range of 100,000 to 1,000,000 Saybolt Universal Second (SUS) units at 38°C (100°F) and a thermal conductivity less than 0.1 W/(m.°C).

While mainly useful for man, the passive heating pad may be used with other animals, such as horses.

In this invention, the passive heating pad comprises an envelope or a plurality of envelopes made from a plastic material which is impermeable to water and which can be heat sealed. The envelope or envelopes contain a viscous composition which has a thermal conductivity of less than 0.1 W/(m.°C). The viscosity of the composition should be such that the composition is comparatively stiff when cold or cool but flows easily when blood heat is reached. The composition has a viscosity in the range of 100,000 to 1,000,000 Saybolt Universal Second (SUS) units at 38°C (100°F) (approximately body heat temperature). The pad also may be made of a plastic envelope into which is placed a rubber or rubber-like formulation, which has a low thermal conductivity, and which can be cut to fit the envelope.

In use, the passive heating pad is placed over the muscle, muscle group or joint where relief is required and is held there by a bandage. The skin below the pad commences to heat up, since the normal radiation of heat from the skin is inhibited by the pad. If the muscle or muscle group is exercised, the heat release is increased until perspiration commences.

The evaporation of the perspiration is inhibited as it is beneath the pad and the pad is in contact engagement with the skin and, therefore, the muscle or group below the pad increases in temperature. In order to maximize this effect, the pad moulds itself closely to the skin surface to inhibit the evaporation of the perspiration.

Increased heat in the muscles has the effect of increasing the blood flow, not only in the area under the pad, but also in adjacent areas. Increased blood flow has a salutary and healing effect on numerous minor injuries which can occur in the human frame, either at work or at play.

In one preferred embodiment of the invention, the high viscosity material comprises a three-component system comprising a liquid polybutene, a petroleum wax and a solid filler material. In this composition, the polybutene component acts as a high viscosity matrix material, and the petroleum wax acts as a skeleton around which the polybutene flows.

Polybutene is available in a variety of grades and molecular weights from various manufacturers. A liquid polybutene having a number average molecular weight in the range of 1,000 to 1,500 is preferred. One such polybutene is that available from Ashland Chemicals under the trademark "Petrofin 300", which has a number average molecular weight of 1390. Polybutenes usually have a high viscosity at body heat temperatures but a much lower viscosity at higher values.

The presence of the petroleum wax and the solid filler material in the composition increases the stiffness of the mixture to provide a higher viscosity value, within the viscosity range of 100,000 to 1,000,000 SUS units at 100°F (38°C) (i.e., approximately body heat temperature).

The solid filler material may be a finely-divided inert solid, such as calcium carbonate, sawdust or powdered rubber.

Essential to this invention is the use of a material having a thermal conductivity of less than 0.1 W/(m.°C). If the thermal conductivity is higher, more heat is lost through the pad by the muscle or muscle group that is being covered by it and the muscles do not heat up fast enough for the protection from injury or healing effect on muscles or joints that the increased blood flow accords. The thermal conductivity value of less than 0.1 W/(m.°C) used herein is a lot lower than that of many common materials, such as water (0.594 W/(m.°C)), ethylene or propylene glycol and their mixtures with water (greater than 0.265 W/(m.°C)), Vaseline (0.183 W/(m.°C)), sand (0.33 W/(m.°C)), glycerol (0.284 W/(m.°C)), and natural rubber (0.138 W/(m.°C)). None of these materials, therefore, is suitable for use in a passive heating pad.

In a preferred embodiment of the heating pad, the filler material preferably is formulated so as to generate some heat by internal friction, such as by the utilization of finely divided solids, for example, sawdust or powdered rubber, dispersed in a viscous medium.

The passive heating pad of the invention is distinguished from the prior art in providing passive heat therapy, while in the prior art some form of external heat needs to be applied to a medium to be effective. There have, however, been a few attempts at providing a passive heating pad but none has the utility of the pad of this invention.

For example, US-A-2,911,974 describes a device which surrounds an entire body member and elastic means is used to maintain and seal the bag to the limb. The bag has no insulating qualities in itself and external insulation is provided to maintain heat localized. The described structure cannot be adapted to cover a single muscle or group of muscles.

Similarly, in US-A-2,909,176, there is described a pad structure comprising a fabric lining and insulating batting which is arranged about an entire body member. Again, the structure cannot be adapted to cover a single muscle or group of muscles.

Further, in the case of both these patents, the structure is not able to absorb heat, so that upon ceasing exercising and working, the heat pad loses its heat very quickly to the ambient air. As a result of the use of low conductivity material in the present invention, heat is stored and available to continue heating the muscle or group of muscles once the exercise has finished.

US-A-3,901,225 describes the use of an air bladder to force a therapeutic pad into pressure contact with an injured area. There are no specific materials specified for the therapeutic pad but it is clear that, for heating purposes, the materials are preheated.

US-A-3,900,035 has been described above. The use of sand as a heat-retaining material in a therapeutic elastic bandage is described. However, the sand is preheated and, in any event, has a thermal conductivity well above the maximum value used in the passive heating pad of the invention.

US-A-3,463,161 describes a heating pad which is described as including a heat maintaining composition. The material is preheated to the required temperature prior to application to the required treatment area. The materials described as suitable in this prior art have thermal conductivities well above the maximum value of 0.1 W/(m.°C) used herein.

US-A-2,595,328 describes a hot pack type of material and forms the preamble of Claim 1. This prior art mentions, as filler materials, aqueous solutions of alcohol, glycerol and ethylene glycol, as well as gases and small solid particles, such as sand, ground cork, soapstone, resilient rubber, diatomaceous earth or mixtures of the same. Ground cork and diatomaceous earth have thermal conductivities of less than 0.1 W/(m.°C) as indeed do most gases. However, since they are used as small particles in an air medium, heat can leak readily through the device and the heat retention capability of the heating pad of the invention is not readily achieved.

The envelope material of the heating pad may be constructed of heat-sealable nylon material or any other impervious plastic material which is hoot sealable and inert to living matter. The envelope is filled with the filler material and a plurality of envelopes may be arranged in a sheet with tear lines surrounding each, so that a pad of any desired size comprising a single or a multiple number of envelopes may be provided.

## Claims

1. A passive heating pad comprising at least one flexible generally planar envelope dimensioned to engage an area of skin adjacent muscle tissue to be treated and flexible to snugly engage the skin area, characterized in that the at least one envelope is filled with a permanently-liquid material having a viscosity in the range of 100,000 to 1,000,000 Saybolt Universal Second (SUS) units at 38°C (100°F) and a thermal conductivity of less than 0.1 W/m.°C).

2. A heating pad as claimed in Claim 1, wherein the pad is provided as one of a sheet of identical heating pads arranged with tear lines between individual pads to permit the provision of single of multiple pad units for a desired end use.

3. A heating pad as claimed in Claim 1 or Claim 2, wherein the liquid material has a decreasing viscosity with increasing temperature.

4. The use of a liquid material having a viscosity in the range of 100,000 to 1,000,000 Saybolt Universal Second (SUS) units at 38°C (100°F) and a thermal conductivity of less than 0.1 W/(m.°C) in the manufacture of a flexible generally planar passive heating pad for the heat treatment of muscle or a group of muscles.

5. A use as claimed in Claim 4, wherein the material comprises a continuous liquid phase and finely-divided solid filler material dispersed in said liquid phase.

6. A use as claimed in Claim 5, wherein said material comprises liquid polybutene, a petroleum wax and said filler material.

7. As use as claimed in Claim 6, wherein said polybutene has a number average molecular weight of 1,000 to 1,500.

8. A use as claimed in Claim 6 or Claim 7, wherein said solid filler material is calcium carbonate.

9. A use as claimed in any one of Claims 5 to 7, wherein said solid filler material is sawdust or powdered rubber.

10. A use as claimed in any one of Claims 4 to 9, wherein the liquid material has a decreasing viscosity with increasing temperature.

11. A use as claimed in any one of Claims 4 to 10, wherein the pad is for the treatment of arthritis.

## Patentansprüche

1. Ein passives Heizkissen mit wenigstens einer flexiblen, im wesentlichen planen Hülle, die so bemessen ist, daß sie eine Hautfläche neben zu behandelndem Muskelgewebe umgreift und so beweglich ist, daß sie an der Hautfläche passend anliegt, dadurch gekennzeichnet, daß die wenigstens eine Hülle mit einem dauernd flüssigen Material gefüllt ist, das eine Viskosität von 100.000 bis 1.000.000 Saybolt Universal Sekunden (SUS) Einheiten bei 38°C (100°F) und eine thermische Leitfähigkeit von weniger als 0,1 W/(m·°C) hat.

2. Ein Heizkissen nach Anspruch 1, bei dem das Kissen als eines einer Lage identischer Heizkissen vorgesehen ist, die mit Reißlinien zwischen einzelnen Kissen versehen sind, um das Vorsehen einzelner oder mehrfacher Kisseneinheiten für einen gewünschten Endzweck zu ermöglichen.

3. Ein Heizkissen nach Anspruch 1 oder 2, bei dem das flüssige Material eine mit ansteigender Temperatur abnehmende Viskosität hat.

4. Die Verwendung eines flüssigen Materials mit einer Viskosität im Bereich von 100.000 bis 1.000.000 Saybolt Universal Sekunden (SUS) Einheiten bei 38°C (100°F) und einer thermischen Leitfähigkeit von weniger als 0,1 W/(m·°C) bei der Herstellung eines flexiblen im wesentlichen ebenen passiven Heizkissens für die Wärmebehandlung eines Muskels oder einer Gruppe von Muskeln.

5. Eine in Anspruch 4 beanspruchte Verwendung, bei der das Material eine kontinuierliche Flüssigphase und ein fein verteiltes festes Füllmaterial aufweist, das in der Flüssigphase dispergiert ist.

6. Eine in Anspruch 5 beanspruchte Verwendung, bei der das Material flüssiges Polybuten, ein Petroleumwachs und das Füllmaterial aufweist.

7. Eine in Anspruch 6 beanspruchte Verwendung, bei der das Polybuten eine durchschnittliche Molekulargewichtszahl von 1.000 bis 1.500 hat.

8. Eine in Anspruch 6 oder Anspruch 7 beanspruchte Verwendung, bei der das feste Füllmaterial Calciumcarbonat ist.

9. Eine in einem der Ansprüche 5 bis 7 beanspruchte Verwendung, bei der das feste Füllmaterial Sägemehl oder pulverisierter Gummi ist.

10. Eine in einem der Ansprüche 4 bis 9 beanspruchte Verwendung, bei der das flüssige Material eine bei ansteigender Temperatur abnehmende Viskosität hat.

11. Eine in einem der Ansprüche 4 bis 10 beanspruchte Verwendung, bei der das Kissen zur Behandlung von Arthritis dient.

## Revendications

1. Tampon de chauffage passif comprenant au moins une enveloppe flexible, généralement planaire, dimensionnée pour occuper une surface de peau en position adjacente au tissu musculaire à traiter et flexible pour occuper de manière confortable la surface de peau, caractérisé en ce qu'au moins une enveloppe est remplie par une matière liquide en permanence, possédant une viscosité de 100 000 à 1 000 000 d'unités en secondes universelles de Saybolt (SUS) à 38°C (100°F) et une conductivité thermique inférieure à 0,1 W/(m.°C).

2. Tampon de chauffage selon la revendication 1, dans lequel on fournit le tampon sous forme d'une feuille constituée de tampons de chauffage identiques munis de lignes de partage entre les tampons individuels pour pouvoir fournir une unité unique de tampon constituée de plusieurs unités de tampon en fonction d'une utilisation finale souhaitée.

3. Tampon de chauffage selon la revendication 1 ou la revendication 2, dans lequel la viscosité de la matière liquide décroît lorsque la température croît.

4. Utilisation d'une matière liquide possédant une viscosité de 100 000 à 1 000 000 d'unités en secondes universelles de Saybolt (SUS) à 38°C (100°F) et une conductivité thermique inférieure à 0,1 W/(m.°C) dans la production d'un tampon de chauffage passif, flexible, généralement planaire, destiné au traitement thermique d'un muscle ou d'un ensemble de muscles.

5. Utilisation selon la revendication 4, dans laquelle la matière comprend une phase liquide continue et une matière de charge solide finement divisée dispersée dans ladite phase liquide.

6. Utilisation selon la revendication 5, dans laquelle ladite matière comprend du polybutène liquide, une cire de pétrole et ladite matière de charge.

7. Utilisation selon la revendication 6, dans laquelle ledit polybutène a une masse moléculaire en nombre de 1000 à 1500.

8. Utilisation selon la revendication 6 ou la revendication 7, dans laquelle ladite matière de charge solide est le carbonate de calcium.

9. Utilisation selon l'une quelconque des revendications 5 à 7, dans laquelle ladite matière de charge solide est de la sciure ou du caoutchouc pulvérulent.

10. Utilisation selon l'une quelconque des revendications 4 à 9, dans laquelle la viscosité de la matière liquide décroît lorsque la température croît.

11. Utilisation selon l'une quelconque des revendications 4 à 10, dans laquelle le tampon est destiné au traitement d'arthrite.
